# EUROPÄISCHE PATENTSCHRIFT

(11) **EP 1 063 998 B1**
(45) Veröffentlichungstag und Bekanntmachung des Hinweises auf die Patenterteilung: **15.01.2003**
(21) Anmeldenummer: 99913218.6
(22) Anmeldetag: 03.03.1999
(51) Int. Cl.: A61K 31/565, A61P 35/02

(54) **MEDIKAMENT ZUR PROPHYLAXE UND/ODER BEHANDLUNG DES MAMMARKARZINOMS ENTHALTEND EINEN STEROIDALEN AROMATASEINHIBITOR**
MEDICAMENT FOR PREVENTING AND/OR TREATING A MAMMARY CARCINOMA, CONTAINING A STEROIDAL AROMATASE INHIBITOR
MEDICAMENT CONTENANT UN INHIBITEUR STEROIDIEN DE L'AROMATASE POUR LA PROPHYLAXIE ET/OU LE TRAITEMENT DU CARCINOME DU SEIN

(30) Priorität: 18.03.1998 EP 98104949
(43) Veröffentlichungstag der Anmeldung: 03.01.2001
(73) Patentinhaber: S.W. Patentverwertungs GmbH, 5026 Salzburg (AT)
(72) Erfinder: SCHMIDT, Alfred, D-22529 Hamburg (DE); WIELAND, Heinrich, D-79271 St. Peter (DE)
(74) Vertreter: Glawe, Delfs, Moll & Partner
(86) Internationale Anmeldenummer: EP9901374
(87) Internationale Veröffentlichungsnummer: WO99047143

(56) Entgegenhaltungen:
- EP-A- 0 310 542
- WO-A-85/03228
- WO-A-92/10482
- WO-A-93/25548
- WO-A-96/08231
- WO-A-97/36570
- BRODIE A M: "Inactivation of aromatase in vitro by 4-hydroxy-4-androstene-3,17-dione and 4-acetoxy-4-androstene-3,17-dione and sustained effects in vivo" STN INTERNATIONAL, KARLSRUHE. FILE MEDLINE, AN=82154103, XP002104191 & STEROIDS, (1981 DEC) 38 (6) 693-702. ,
- BRODIE A M: "Overview of recent development of aromatase inhibitors." STN INTERNATIONAL, KARLSRUHE. FILE MEDLINE, AN=82208293 , XP002104192 & CANCER RESEARCH, (1982 AUG) 42 (8 SUPPL) 3312S-3314S,

## Beschreibung

Die Erfindung betrifft die Verwendung eines steroidalen Aromataseinhibitors zur Herstellung eines Medikaments zur Prophylaxe (Primärund Sekundärprophylaxe) und/oder Behandlung des Mammakarzinoms.

Das Mammakarzinom (Brustkrebs) ist bei Frauen die häufigste maligne Erkrankung. In Deutschland macht der Brustkrebs etwa 20% aller Krebsfälle bei Frauen aus, die Inzidenz liegt derzeit bei über 30.000 Neuerkrankungen pro Jahr. Die heute angewendete adjuvante Krebstherapie führt zwar zu einer Steigerung der Überlebensrate, auch kann Brustkrebsscreening und frühe chirurgische Behandlung die Mortalität um über 30% senken. Da jedoch die Anzahl der Neuerkrankungen ständig zunimmt, bleibt die Todesrate gemessen an der Gesamtbevölkerung gleich oder steigt. Die Zahl der Neuerkrankungen läßt sich bisher kaum beeinflussen, da zuwenig über auslösende Faktoren bekannt ist.

Bei etwa der Hälfte aller Mammakarzinome finden sich Östrogen- und/oder Progesteronrezeptoren im Zytoplasma. Solche Mammakarzinome benötigen für die Proliferation ihrer Zellen Östrogen. Östrogene wirken durch Bindung an spezifische intrazelluläre (zytoplasmatische) Rezeptoren von östrogenempfindlichen Zellen, in die sie passiv durch Diffusion aus dem Plasma eingeschleust werden. Die Bindung verändert die Konfiguration des Rezeptorproteins. Der Rezeptor-Hormon-Komplex steuert sowohl die Transkription als auch die Expression spezifischer Gene, die dadurch bewirkte Synthese von wachstumsfördernden und/oder wachstumshemmenden Faktoren wirkt letztlich auf das Zellwachstum ein.

Durch Entzug von Östrogenen kann eine Rückbildung östrogenabhängiger Tumoren bewirkt werden. Bei prämenopausalen Frauen sind die Ovarien Hauptquelle von Östrogenen. Deren chirurgische Entfernung wird daher bereits seit 1896 bei Brustkrebs im fortgeschrittenen Stadium (Metastasenbildung) als sogenannte chirurgische Hormontherapie durchgeführt.

Bei postmenopausalen Frauen ist die Umwandlung adrenaler Androgene, vor allem Androstendion und Testosteron, zu Östron und Östradiol die Hauptöstrogenquelle. Die Umwandlung zu Östrogenen findet im Muskel- und Fettgewebe statt.

In der klinischen Praxis werden seit über zwanzig Jahren sowohl frühe als auch fortgeschrittene Stadien des Mammakarzinoms mit Tamoxifen oder dessen Derivaten (insbesondere Tamoxifencitrat) behandelt. Tamoxifen belegt die im Zytoplasma der Krebszellen vorhandenen Östrogenrezeptoren und bewirkt so eine kompetitive Verdrängung von Östrogenen. Der aus Tamoxifen und dem Östrogenrezeptor gebildete Komplex verhindert die ansonsten von einem Komplex aus Östrogenen und dem Rezeptor bewirkte Transkription und Expression der das Zellwachstum fördernden Gene.

Bei in vitro-Versuchen ist nachgewiesen worden, daß Tamoxifen auch auf Zellinien wachstumshemmend und unter Umständen sogar zytostatisch wirkt, die über keine Östrogenrezeptoren verfügen. Tamoxifen hemmt die Proteinkinase C und blockiert die Aktivierung von Calmodulin. Es steigert die Aktivität der Killerzellen und hemmt Suppressor T-Lymphozyten.

Insbesondere nach längerer Behandlung kann Tamoxifen auf eine nicht näher bekannte Art und Weise auf Krebszellen auch wie Östrogene wirken und deren Wachstum fördern. Eine länger andauernde Tamoxifen-Behandlung kann daher u.U. zu einem Tumorwachstum führen. Sie führt zudem zu einem um den Faktor 3 bis 5 erhöhten Risiko, an Krebs der Gebärmutterschleimhaut zu erkranken. Angesichts des klinischen Nutzens der Tamoxifen-Therapie nimmt man dieses Risiko bei Brustkrebspatientinnen in Kauf.

Bekannt ist ferner die systemische Behandlung von Mammakarzi-nomen mit Aromataseinhibitoren, insbesondere 4-Hydroxyan-drost-4-en-3,17-dion (INN Formestan). Die Aromatase ist ein komplexes Enzymsystem, das die Umwandlung adrenaler Androgene zu Östron und Östradiol katalysiert.

Formestan ist ein Derivat des physiologischen Stereoidhormons Androstendion und bindet kompetetiv zu anderen Substraten an Aromatase. Es schädigt während der Katalyse das Enzymmolekül irreversibel. Die systemische Behandlung mit Formestan wird ebenfalls als antiöstrogene Brustkrebstherapie angewandt.

WO-A-93/25548 offenbart die Herstellung von Triazol-Isobenzofuran-Derivaten und deren Verwendung als Aromataseinhibitoren. WO-A-92/10482 offenbart Aromataseinhibitoren, die funktionalisierte Vinylazole enthalten. Beide Schriften offenbaren lediglich die systemische Administration solcher Aromataseinhibitoren, d. h. es soll zur Erzielung der gewünschten Wirkung ein Blutserumspiegel des Wirkstoffes eingestellt werden.

Der Erfindung liegt die Aufgabe zugrunde, ein Medikament der eingangs genannten Art zu schaffen, das für eine Behandlung und insbesondere auch Prophylaxe des Mammakarzinoms geeignet ist.

Die Erfindung löst diese Aufgabe dadurch, daß ein steroidaler Aromataseinhibitor zu einem für die lokale topische Applikation vorgesehenen Medikament formuliert wird. Die Verwendung von Antigestagenen als zusätzlicher Bestandteil des Medikaments ist im Rahmen der Erfindung ausgeschlossen.

Zunächst seien einige im Rahmen der Erfindung verwendete Begriffe erläutert.

Erfindungsgemäß werden steroidale Aromataseinhibitoren als die Bildung von Östrogenen inhibierende Substanzen verwendet. Diese inhibieren die Östrogenbiosynthese aus den androgenen Vorstufen, bspw. die enzymatische Umwandlung von Androstendion zu Östron oder von Testosteron zu Östradiol. Da die beiden genannten Syntheseschritte durch das Enzymsystem der Aromatase (converting enzyme) vermittelt werden, werden Aromataseinhibitoren im Rahmen der Erfindung verwendet. Bevorzugt sind Inhibitoren, die an die Aromatase binden und diese irreversibel schädigen. Nach der topischen Applikation penetrieren sie durch die Haut und reichern sich im Fettgewebe an.

Das erfindungsgemäße Medikament ist für die lokale topische Applikation vorgesehen. Das Medikament wird lokal auf die Haut aufgetragen, der vorzugsweise stark lipophile Wirkstoff wird transdermal resorbiert und somit lokal an den vorgesehenen Wirkort gebracht. Der Wirkstoff reichert sich im periduktalen Fettgewebe an. Bei einer Langfristbehandlung wird die Fettmasse der behandelten Brust deutlich reduziert. Diese Reduktion vermindert die Menge der Östrogenbildnerzellen mit Östrogenbildner-Kompetenz. Die Lipophilie und Hydrophobie des Wirkstoffs hat zur Folge, daß sich der Wirkstoff ausschließlich lokal im Fettgewebe anreichert und keine systemische Wirkung entfalten kann. Unter Östrogenen sind alle weiblichen Sexualhormone mit einer Wirkung vergleichbar bspw. der von Östron und Östradiol zu verstehen.

Bei der aus dem Stand der Technik bekannten Anwendung von Aromataseinhibitoren wie Formestan ist vorgesehen, diese über den Blutkreislauf zum Wirkort zu transportieren. Man strebt hohe Serumkonzentrationen des Aromataseinhibitors an, die neben der gewünschten Wirkung im Tumor zu systemischen Nebenwirkungen führen können. Angesicht der Schwere der Krankheit werden solche Nebenwirkungen in der Akuttherapie im Hinblick auf den gewünschten Erfolg in Kauf genommen. Dies kommt jedoch nicht in Betracht im Fall einer vorbeugenden Behandlung gegen eine noch nicht bestehende bzw. eindeutig nachgewiesene Erkrankung.

Erfindungsgemäß ist hingegen vorgesehen, das Medikament topisch unmittelbar am oder in der Nähe des vorgesehenen Wirk-ortes zu applizieren. Anders als im Stand der Technik wird kein Transport über den Blutkreislauf an dem vorgesehenen Wirkort angestrebt. Erfindungsgemäß wird erreicht, daß ein hinreichender Wirkstoffspiegel im Risikogewebe (bei der Prophylaxe) bzw. im erkrankten Gewebe (bei der Therapie) lokal entsteht, ohne daß eine nennenswerte Resorption des Wirkstoffs in dem Blutkreislaufs stattfindet. Der Kern der Erfindung liegt also nicht nur in der topischen Applikation an sich, sondern in der lokalen topischen Applikation dergestalt, daß der Wirkstoff sich unmittelbar und nicht über den Umweg des Blutkreislauf im Risikogewebe und/oder erkrankten Gewebe anreichert.

Wenn auch metastasierende Karzinome behandelt und/oder gegen diese vorgebeugt werden soll, kann das erfindungsgemäße Medikament topisch in einer solchen Menge am vorgesehenen Wirkort appliziert werden, daß zusätzlich eine nennenswerte Resorption in dem Blutkreislauf stattfindet und sich somit ein Serumspiegel aufbaut, der den Wirkstoff auch zu Metastasen transportiert. Auch bei dieser Anwendung erfolgt primär eine lokale Resorption am oder in der Nähe des vorgesehenen Hauptwirkortes.

Das Medikament kann zur Behandlung des Mammakarzinoms dienen. Diese Behandlung kann nach einer chirurgischen Primärversorgung und ggf. entsprechender adjuvanter Therapie die bisher übliche systemische antiöstrogene Therapie ersetzen und/oder ergänzen.

Ein wichtiger Vorteil der Erfindung ist die Möglichkeit, das Medikament auch für die Brustkrebsprophylaxe einzusetzen. Eine besonders vorteilhafte Einsatzmöglichkeit ist die sogenannte Sekundärprophylaxe. Bei Patientinnen, bei denen bereits ein Mammakarzinom vorliegt, besteht ein besonders hohes Risiko für ein weiteres Karzinom in der kontralateralen Brust. Die kontralaterale Brust kann dann mit dem erfindungsgemäßen Medikament prophylaktisch behandelt werden. Ebenfalls möglich ist eine sekundärprophylaktische Behandlung der erkrankten Brust zur Vermeidung lokaler Rezidive.

Bei sogenannten Hochrisikofrauen kann eine Primärprophylaxe vorgenommen werden. Als Auswahlkriterien für eine solche Hochrisikogruppe können bspw. die Tatsachen dienen, daß mindestens eine Verwandte ersten Grades mütterlicherseits entweder vor dem 45. Lebensjahr oder beidseitig an Brustkrebs erkrankt ist, oder daß mütterlicherseits mindestens eine Verwandte ersten Grades und eine zusätzliche Verwandte an Brustkrebs erkrankt sind. Da die erfindungsgemäße lokale Applikation mögliche systemische Nebenwirkungen des Wirkstoffes aufgrund dessen Hydrophobie praktisch vollständig vermeidet, kann die Indikation zur Primärprophylaxe relativ großzügig bereits bei Vorliegen von Gewebe mit verhältnismäßig geringem oder mittlerem Risiko (bspw. Histologischer Befund Prechtel II oder III) gestellt werden. Mit der Prophylaxe kann begonnen werden, auch wenn die herkömmliche Frühdiagnostik (Palpationsbefund) noch negativ ist. Denn diese übliche Frühdiagnostik ist unzureichend und erkennt ein Mammakarzinom in der Regel erst dann wenn bereits eine kaum noch curable systemische Erkrankung vorliegt.

Das erfindungsgemäße Medikament wird vorzugsweise über einen längeren Zeitraum (bei Bedarf bis lebenslang) lokal und topisch appliziert, die Applikation erfolgt bspw. ein- bis zweimal pro Tag.

Die Wirksubstanzen werden aus der Gruppe der (bevorzugt lipidlöslichen) steroidalen Aromataseinhibitoren ausgewählt. Diese lipidlöslichen Substanzen dringen bei topischer Applikation in das Fettgewebe ein und behindern lokal die de-novo-Bildung von Östrogenen aus den Östrogenvorstufen.

Verwendbar sind bspw. steroidale Aromataseinhibitoren wie 4-Hydroxyandrost-4-en-3,17-dion (Formestan), 6-Methylenandrostra-1,4-dien-3,17-dion (Exemestan), 10-(2-Propynyl)ester-4-en-3,17-dion (MDL 18962) und 7-α-substituierte Androstendionderivate.

Bei den in Klammern genannten Bezeichnungen handelt es sich um die INN (International nonproprietary names). Zur Terminologie und Struktur der genannten Substanzen wird gleichfalls auf die Rote Liste sowie Römpp Chemielexikon verwiesen.

Die genannten Substanzen sind bisher nur für die systemische Therapie von Brustkrebs verwendet worden. Erfindungsgemäß wird dagegen der Wirkstoff durch lokale Applikation an den vorgesehenen Wirkort gebracht. Bei der Verwendung von Aromataseinhibitoren erreicht die Erfindung eine Verminderung der Aromataseaktivität im Fettgewebe der Brust, also genau an der Stelle, an der ein Tumor entstehen oder wachsen kann. Bei längerfristiger Anwendung wird die Fettmasse der Brust und damit die Menge an möglichem Risikogewebe reduziert. Da Mammakarzinome häufig in oberen Brustquadranten erhöhter Aromataseaktivität entstehen, ist dort erfindungsgemäß eine besonders wirkungsvolle Prophylaxe möglich.

Die erfindungsgemäße Verwendung von Aromataseinhibitoren kann prophylaktisch oder therapeutisch auch gegen solche Tumore der Mamma eingesetzt werden, die selbst in der Lage sind, Östrogen für autokrine/parakrine Stimulation zu produzieren. Eine Senkung der Östrogenkonzentration im Plasma wirkt sich auf solche Tumoren kaum aus, die erfindungsgemäß zu erzielende Verminderung der intratumoralen Aromatasekonzentration aufgrund der Verwendung zellgängiger Inhibitoren kann jedoch solche Tumore beeinflussen.

Da die erfindungsgemäß zugeführten Wirkstoffe aufgrund ihrer Lipidlöslichkeit im Fettgewebe der Brust lokalisiert bleiben und dort ihre vorgesehene Wirkung entfalten, scheiden die durch eine systemische Applikation induzierten Nebenwirkungen aus. Diese Verminderung bzw. Ausschaltung von Nebenwirkungen erlaubt eine wesentlich breitere prophylaktische Anwendung. Das erfindungsgemäße Medikament kann von Patientinnen selbst appliziert werden, häufige Arztbesuche zu diesem Zweck sind nicht erforderlich.

Bevorzugt enthält ein erfindungsgemäß formuliertes Medikament Formestan.

Formestanderivate wie bspw. acetyliertes Formestan (bspw. 4-O-Acetylandrost-4-en-3,17-dion) sind ebenfalls bevorzugt verwendbar. Die Acetylierung des Formestans erhöht dessen Hydrophilie und damit Hautgängigkeit (skin penetration) wesentlich. Da die Acetylgruppe nach dem Durchtritt durch die Haut unter den im subkutanen Bereich herrschenden Bedingungen abhydrolisiert wird, entsteht in situ wieder der eigentliche Wirkstoff Formestan. Bei der Verwendung eines solchen acetylierten Formestans appliziert man somit eine besser durch die Haut tretende Vorstufe des eigentlichen Wirkstoffs, die Erfinder haben erkannt, daß aus dieser Vorstufe der eigentliche Wirkstoff subkutan in situ entsteht.

Die erfindungsgemäß verwendeten Wirkstoffe sind in der Regel lipidlöslich und eignen sich gut für eine topische Applikation. Wie oben bereits beschrieben, vermeidet die Anreicherung im Fettgewebe der Brust systemische Nebenwirkungen. Zur Verbesserung der Hautgängigkeit können dem erfindungsgemäßen Medikament im Stand der Technik bekannte Stoffe hinzugefügt werden, die diese fördern, bspw. Hyaluronidate oder DMSO (Dimethylsulfoxid).

Das Medikament ist vorzugsweise als Salbe, Creme, Gel, Emulsion oder Lotion formuliert. Eine Formulierung als Puder oder Öl ist ebenfalls denkbar. Formulierungsgrundlagen sind dem Fachmann aus der kosmetischen und pharmazeutischen Industrie geläufig und brauchen hier nicht näher erläutert zu werden. Verwendbar sind bspw. pflanzliche Öle und Fette wie Mandelöl, Erdnußöl, Olivenöl, Pfirsichkernöl, Rizinus- bzw. Castoröl, Pflanzenextrakte, etherische Öle; ferner pflanzliche Wachse sowie synthetische und tierische Öle, Fette oder Wachse; Lecithin, Lanolinalkohole, Karotin, Duftstoffe, ein- oder mehrwertige Alkohole, Harnstoff, Konservierungs- und Farbstoffe usw. Bevorzugt ist eine Formulierung als Öl-in-Wasser- oder Wasser-in-Öl-Emulsion.

Der Wirkstoffgehalt des Medikaments (der Gehalt an die Bildung von Östrogenen inhibierenden Substanzen) kann zwischen 0,0001 und 20 Gew.-%, vorzugsweise 0,6 und 10 Gew.-%, weiter vorzugsweise 1 und 5 Gew.-% liegen. Ein üblicher Bereich ist 0,6 bis 2 Gew.-%.

Sofern Stoffe zur Förderung der Hautgängigkeit Resorption beigemischt werden, kann deren Gehalt bei der Verwendung von Hyaluronidasen bspw. zwischen 0,01 und 1 Gew.-%, vorzugsweise 0,05 und 0,2 Gew.-% liegen; im Fall der Verwendung von DMSO zwischen 1 und 25 Gew.-%, vorzugsweise 5 und 10 Gew.-%.

Ausführungsformen der Erfindung werden nachfolgend beschrieben. In der Zeichnung zeigen:
- Fig.1: den zytologischen Befund eines Fettzell-Aspirats vor erfindungsgemäßer Anwendung des Medikaments,
- Fig.2: den entsprechenden Befund nach täglicher Anwendung über 3 Monate.

### Beispiel 1

Die folgenden Bestandteile wurden zu einer Creme vermischt:

| | |
|---|---|
| Harnstoff | 10 g |
| Titanoxid | 15 g |
| Rohvaseline | 20 g |
| Isopropylpalmitat | 10 g |
| gehärtetes Erdnußöl | 10 g |
| Tween 80 | 5 g |
| Formestan | 1 g |
| mit gereinigtem Wasser aufgefüllt auf | 100 g |

### Beispiel 2

Ein Gel wurde aus folgenden Bestandteilen hergestellt:

| | |
|---|---|
| Ethanol 90% | 7,0 g |
| Carbopol^{R} 934 P | 7,0 g |
| Triethanolamin | 2 g |
| Polysorbat 80 | 5,0 g |
| Glycerol | 3,0 g |
| Formestan | 0,75 g |
| gereinigtes Wasser auf | 100 g |

### Beispiel 3

Eine Creme wurde aus folgenden Bestandteilen hergestellt:

| | |
|---|---|
| Propylenglykol | 25,0 g |
| Isopropylmyristat | 6,0 g |
| Sorbitanmonostearat | 1,0 g |
| Polysorbat 80 | 2,0 g |
| Cetylstearylalkohol | 6,0 g |
| Stearylalkohol | 2,0 g |
| Glycerolmonostearat | 1,0 g |
| Hyaluronsäure | 0,1 g |
| Formestan | 1,5 g |
| gereinigtes Wasser auf | 100 g |

### Beispiel 4

Eine Creme wurde aus den nachfolgenden Bestandteilen hergestellt. Die Bestandteile sind bei diesem Beispiel mit ihren INCI-Namen angegeben.

| INCI | |
|---|---|
| Ceteareth-25 | 3,0 g |
| PEG-4-Polyglyceryl-2-Stearate | 2,0 g |
| Cetearyl Alcohol | 4,9 g |
| Petrolatum | 10,0 g |
| Paraffinum Perliquidum | 3,0 g |
| Sodium Carbomer 400 | 0,14 g |
| Lactic Acid | 0,02 g |
| Paraffinum Perliquidum | 2,0 g |
| Phenoxyethanol, Dehydroacetic Acid, Benzoic Acid | 0,4 g |
| Parfum | 0,08 g |
| Formestan | 1,5 g |
| aufgefüllt mit Wasser auf | 100 g |

Die in der Formulierung genannte Mischung aus Phenoxyethanol, Dehydroessigsäure und Benzoesäure ist von der Firma Schülke & Mayr unter dem Namen Euxyl^{R} K702 erhältlich.

### Beispiel 5

Eine Creme wurde aus den nachfolgenden Bestandteilen hergestellt, die mit ihren INCI-Namen angegeben sind.

| INCI | |
|---|---|
| Ceteareth-25 | 3,0 g |
| PEG-4-Polyglyceryl-2-Stearate | 2,0 g |
| Cetearyl Alcohol | 4,9 g |
| Petrolatum | 10,0 g |
| Paraffinum Perliquidum | 3,0 g |
| Sodium Carbomer 400 | 0,14 g |
| Lactic Acid | 0,02 g |
| Paraffinum Perliquidum | 2,0 g |
| Phenoxyethanol, Dehydroacetic Acid, Benzoic Acid | 0,4 g |
| Parfum | 0,08 g |
| 4-Acetylandrost-4-en-3,17-dion (acetyliertes Formestan) | 1,5 g |
| aufgefüllt mit Wasser auf | 100 g |

### Beispiel 6

Ein klinischer Test der Rezeptur gemäß Beispiel 1 wurde durchgeführt. Bei der 25jährigen Probandin lagen die Befunde übergroße Brüste sowie eine mittelgradige Mastopathie vor.

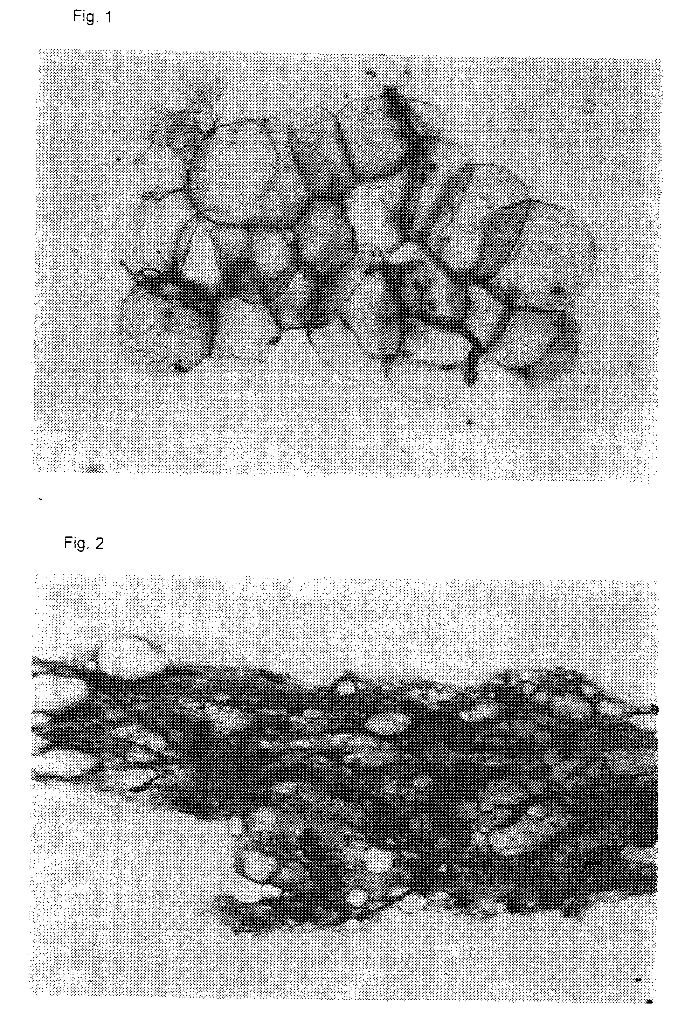

Ein Feinnadel-Aspirat des Fettgewebes (0,6 mm Punktionskanüle, Fixation in absolutem Ethanol, Färbung: May-Grünwald-Giemsa) wurde entnommen (Fig.1) Man erkennt aufgeblähte Fettzellen und exzentrische Zellkerne durch hohen Östrogeneinfluß.

Die Probandin applizierte anschließend die Creme gemäß Beispiel 1 zweimal täglich über einen Zeitraum von 3 Monaten. Fig.2 zeigt das Fettgewebe-Aspirat nach dieser Anwendung. Man erkennt eine durch die Aromatasehemmung bewirkte Volumenreduktion des Fettgewebes ("geschrumpfte" Fettzellen in regelmäßiger Anordnung) sowie eine Bindegewebsvermehrung. Der Befund ergab eine Verminderung des Risikogewebes um ca. 50% sowie eine deutliche Straffung von Bindegewebe und Haut.

## Patentansprüche

1. Verwendung eines steroidalen Aromataseinhibitors zur Herstellung eines für die lokale topische Applikation formulierten Medikaments zur Prophylaxe und/oder Behandlung des Mammakarzinoms, wobei das Medikament keine Antigestagene enthält.

2. Verwendung nach Anspruch 1, **dadurch gekennzeichnet, daß** das Medikament Formestan (4-Hydroxyandrost-4-en-3,17-dion) enthält und/oder ein pharmakologisch wirksames Formestanderivat enthält.

3. Verwendung nach Anspruch 2, **dadurch gekennzeichnet, daß** das Medikament 4-O-Acetylandrost-4-en-3,17-dion enthält.

4. Verwendung nach einem der Ansprüche 1 bis 3, **dadurch gekennzeichnet, daß** das Medikament zusätzlich Stoffe zur Förderung der Hautgängigkeit enthält.

5. Verwendung nach Anspruch 4, **dadurch gekennzeichnet, daß** das Medikament DMSO enthält.

6. Verwendung nach einem der Ansprüche 1 bis 5, **dadurch gekennzeichnet, daß** das Medikament als Salbe, Creme, Gel, Emulsion oder Lotion formuliert ist.

7. Verwendung nach Anspruch 6, **dadurch gekennzeichnet, daß** der Wirkstoffgehalt 0,0001-20 Gew.-%, vorzugsweise 0,6-10 Gew.-%, weiter vorzugsweise 1-5 Gew.-% beträgt.

## Claims

1. Use of a steroidal aromatase inhibitor for the preparation of a medicament formulated for local topical application for the prophylaxis and/or treatment of mastocarcinoma, where the medicament contains no antigestagens.

2. Use according to Claim 1, **characterized in that** the medicament contains formestane
(4-hydroxyandrost-4-ene-3,17-dione) and/or a pharmacologically active formestane derivative.

3. Use according to Claim 2, **characterized in that** the medicament contains 4-O-acetylandrost-4-ene-3,17-dione.

4. Use according to one Claims 1 to 3, **characterized in that** the medicament additionally contains substances for promoting skin penetration.

5. Use according to Claim 4, **characterized in that** the medicament contains DMSO.

6. Use according to one of Claims 1 to 5, **characterized in that** the medicament is formulated as an ointment, cream, gel, emulsion or lotion.

7. Use according to Claim 6, **characterized in that** the active compound content is 0.0001-20% by weight, preferably 0.6-10% by weight, further preferably 1-5% by weight.

## Revendications

1. Utilisation d'un inhibiteur stéroïdien de l'aromatase pour la fabrication d'un médicament formulé pour une application topique locale pour la prophylaxie et/ou le traitement du carcinome du sein, où le médicament ne contient pas d'antiprogestatif.

2. Utilisation selon la revendication 1, **caractérisée en ce que** le médicament contient du formestane (4-hydroxyandrost-4-èn-3,17-dione) et/ou un dérivé de formestane pharmacologiquement actif.

3. Utilisation selon la revendication 2, **caractérisée en ce que** le médicament contient de la 4-O-acétylandrost-4-èn-3,17-dione.

4. Utilisation selon l'une des revendications 1 à 3, **caractérisée en ce que** le médicament contient des substances supplémentaires pour favoriser la perméabilité cutanée.

5. Utilisation selon la revendication 4, **caractérisée en ce que** le médicament contient du DMSO.

6. Utilisation selon l'une des revendications 1 à 5, **caractérisée en ce que** le médicament est formulé sous forme de pommade, crème, gel, émulsion ou lotion.

7. Utilisation selon la revendication 6, **caractérisée en ce que** la teneur en substance active est de 0,0001-20 en % en poids, de préférence de 0,16-10 en % en poids, de préférence de 1-5 en % en poids.
